# EUROPÄISCHE PATENTSCHRIFT

(11) **EP 0 411 252 B1**
(45) Veröffentlichungstag und Bekanntmachung des Hinweises auf die Patenterteilung: **03.11.1993**
(21) Anmeldenummer: 90108226.3
(22) Anmeldetag: 30.04.1990
(51) Int. Cl.: C07C 63/70, C07C 51/29, C07C 49/80, C07C 45/46

(54) **Verfahren zur Herstellung von fluorierten Benzoesäuren**
Process for the preparation of fluorobenzoic acids
Procédé pour la préparation d'acides fluoro-benzoiques

(30) Priorität: 28.07.1989 DE 3925036
(43) Veröffentlichungstag der Anmeldung: 06.02.1991
(73) Patentinhaber: RIEDEL-DE HAEN AKTIENGESELLSCHAFT, D-30926 Seelze 1 (DE)
(72) Erfinder: Schmand, Horst, Dr., D-3052 Bad Nenndorf (DE)
(74) Vertreter: Muley, Ralf, Dr.

(56) Entgegenhaltungen:
- EP-A- 0 176 026
- EP-A- 0 303 291
- EP-A- 0 342 849
- DE-A- 3 529 259
- CHEMICAL ABSTRACTS, Band 85, Nr. 15, 11. Oktober 1976, Columbus, Ohio, USA H. BERCHER et al. "The Synthesis of B-receptor blocking substances" Seite 436, Spalte 1, Zusammenfassung-Nr. 108 367u

## Beschreibung

Die Erfindung betrifft ein Verfahren zur Herstellung von fluorierten Benzoesäuren der Formel I 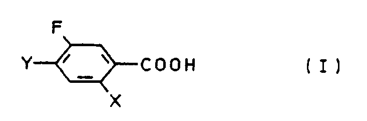
worin
- X =: Br, Cl, F,
- Y =: Cl, F und

einer der Reste X oder Y auch Wasserstoff bedeuten.

Die Verbindungen der Formel I sind bekannt und werden z.B. als Zwischenprodukte zur Herstellung von antibacteriell wirkenden 6-Fluor-chinoloncarbonsäuren eingesetzt (vergl. EP-A2-303 291, Seite 5, Zeilen 25 ff, und EP-A2-342 849, Seite 10, Zeilen 30 ff).

Nach dem aus der EP-A2-303 291 bekannten Verfahren wird 2-Chlor-4,5-difluorbenzoesäure dadurch hergestellt, daß 1-Chlor-3,4-difluorbenzol zu 2-Chlor-4,5-difluoracetophenon mit einer Ausbeute von 82 % der Theorie acyliert und anschließend mit Natriumhypochlorit zu 2-Chlor-4,5-difluorbenzoesäure in einer Ausbeute von 85,1% der Theorie oxidiert wird. Die Gesamtausbeute dieses Verfahrens beläuft sich somit nur auf ca. 70% der Theorie, bezogen auf das 1-Chlor-3,4-difluorbenzol als Ausgangsprodukt. Nachteilig bei diesem Verfahren ist ferner, daß gemäß dem Ausführungsbeispiel die Acylierung bei einer Temperatur von 120°C durchgeführt werden muß.

Nach dem Verfahren der ungeprüften Japanischen Patentpublikation Nr. 108839/1987 wird 2-Chlor-4,5-difluorbenzoesäure aus 2-Chlor-4,5-difluor-benzotrifluorid, und 2,4,5-Trifluor-benzoesäure aus 2,4,5-Trifluor-benzotrifluorid durch Umsetzung mit Schwefelsäure bei Temperaturen von 100 bis 140°C hergestellt. Nachteilig bei diesem Verfahren ist das Freiwerden von Fluorwasserstoff bei Temperaturen über 100°C, wodurch besondere Reaktormaterialien erforderlich werden.

In Beispiel 14 der EP-A2-342 849 wird 1-Brom-3,4-difluorbenzol mit Acetylchlorid und Aluminiumchlorid (Molverhältnis 1 : 1,5 : 2,5) 2 Stunden bei 120°C umgesetzt. Man hydrolisiert überschüssige Reagenzien und den Friedel-Crafts Komplex in Eis und extrahiert dann das Reaktionsprodukt 2-Brom-4,5-difluoracetophenon mit Methylenchlorid. Nach dem Abdampfen des Methylenchlorids wird das 2-Brom-4,5-difluoracetophenon mit überschüssiger industrieller Bleichlauge (Natriumhypochloritlösung) durch zweistündiges Kochen am Rückfluß zur Säure oxidiert. Nach üblicher Aufarbeitung und einer Umfällung erhält man die 2-Brom-4,5-difluorbenzoesäure in einer Ausbeute von 50 % der Theorie. Der Schmelzpunkt beträgt wegen vorhandener Verunreinigungen lediglich 102 bis 104°C.

Aufgabe der vorliegenden Erfindung ist es, ein Verfahren zur Herstellung von Verbindungen der Formel I anzugeben, das die Nachteile der bisherigen Verfahren nicht besitzt. Insbesondere soll das erfindungsgemäße Verfahren die Verbindungen der Formel I unter milderen Bedingungen wirtschaftlicher und in hohen Ausbeuten und Reinheiten liefern.

Es wurde überraschenderweise gefunden, daß fluorierte Chloracetophenone der Formel II 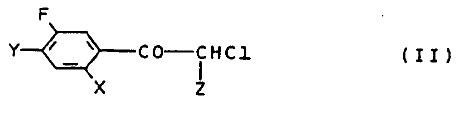
worin
- X =: Br, Cl, F,
- Y =: Cl, F,
- Z =: H oder Cl und

einer der Reste X oder Y auch Wasserstoff bedeuten, durch Umsetzung von Verbindungen der Formel III 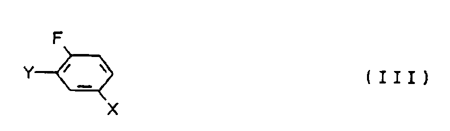
worin
- X =: Br, Cl, F,
- Y =: Cl, F

und einer der Reste X oder Y auch Wasserstoff bedeuten, mit Mono- oder Dichloracetylchorid bei Temperaturen von 20 bis 100°C in hervorragenden Ausbeuten hergestellt und anschließend in hervorragenden Ausbeuten mit einem Hypohalogenit zu den fluorierten Benzoesäuren der Formel I umgesetzt werden können.

Die Erfindung betrifft daher ein Verfahren zur Herstellung der fluorierten Benzoesäuren der Formel I 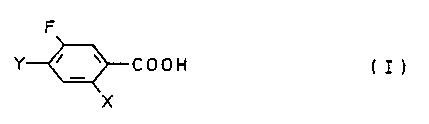
worin
- X =: Br, Cl, F,
- Y =: Cl, F

und einer der Reste X oder Y auch Wasserstoff bedeuten, und ist dadurch gekennzeichnet, daß ein fluoriertes Chloracetophenon der Formel II 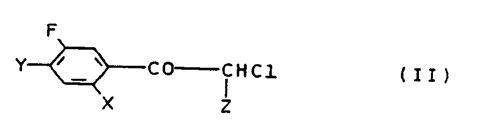
worin
- X =: Br, Cl, F,
- Y =: Cl, F,
- Z =: H oder Cl und

einer der Reste X oder Y auch Wasserstoff bedeuten, mit einem Hypohalogenit umgesetzt wird. Bei dieser Umsetzung wird die Verbindung II zu der entsprechenden Carbonsäure I oxidiert.

Vorzugsweise bedeuten in den Formeln I, II und III Y Fluor und X Fluor oder Chlor, in der Formel II bedeutet darüberhinaus Z vorzugsweise Chlor.

Das Hypohalogenit kann beispielsweise ein Erdalkalimetallhypohalogenit, wie z.B. Calciumhypochlorit, oder insbesondere ein Alkalimetallhypohalogenit, z.B. ein Alkalimetallhypobromit oder Alkalimetallhypochlorit, sein. Als Hypohalogenite werden die Hypochlorite und insbesondere Hypobromite bevorzugt. Weitere Beispiele für geeignete Hypohalogenite sind: Kalium- und Natriumhypochlorit, (z.B. auch in Form der sogenannten Chlorlauge oder Chlorbleichlauge), Kalium- und Natriumhypobromit, von denen das Natriumhypobromit bevorzugt ist.

Das Hypohalogenit kann auch in situ aus dem Halogen, insbesondere Chlor oder Brom, und einer wäßrigen Lösung eines Erdalkalimetall- oder Alkalimetall-hydroxids, insbesondere Kali- oder Natronlauge, erzeugt werden. Als Hypohalogenit kann auch ein Gemisch eines Hypohalogenits mit einem Erdalkalimetall- und/oder Alkalimetall-hydroxid und/oder ein Gemisch verschiedener Hypohalogenite eingesetzt werden.

Die Oxidation der fluorierten Chloracetophenone der Formel II mit dem Hypohalogenit wird zweckmäßigerweise in wäßriger Phase und bei Temperaturen von 0°C bis 100°C, vorzugsweise von 10 bis 80°C und ganz besonders bevorzugt von 20 bis 60°C durchgeführt. Vorzugsweise wird dabei eine wäßrige Lösung des Hypohalogenits vorgelegt und die Verbindung der Formel II zugegeben, insbesondere zugetropft oder zudosiert. Es ist zweckmäßig, bei der Überführung von 1 mol α,α-Dichloracetophenon der Formel II (Z bedeutet dabei in Formel II Chlor) in die Verbindungen der Formel I mindestens 2 mol Hypohalogenit einzusetzen. Für die Überführung von 1 mol Chloracetophenon der Formel II (Z bedeutet dabei in Formel II Wasserstoff) werden zweckmäßigerweise mindestens 3 mol Hypohalogenit eingesetzt. Sofern bei der praktischen Durchführung des erfindungsgemäßen Verfahrens überhaupt gegenüber den genannten Mindestmengen ein Überschuß an Hypohalogenit erforderlich ist, wird zumeist nur ein minimaler Überschuß benötigt.

In manchen Fällen wird bei der Verwendung eines Hypochlorits, z.B. von Natriumhypochlorit, ein größerer Überschuß benötigt als bei der Verwendung eines Hypobromits, z.B. von Natriumhypobromit. Selbstverständlich ist die Verwendung eines größeren Überschusses an Hypohalogenit allgemein möglich, aber nicht erforderlich bzw. aus wirtschaftlichen Gründen nicht zweckmäßig.

Aus dem Reaktionsgemisch können die Verbindungen der Formel I in üblicher Weise, z.B. durch Ausfällen mit einer Mineralsäure, wie z.B. Salzsäure, isoliert werden. Bei dem Ausfällen der Verbindungen der Formel I mit einer Mineralsäure ist es z.B. bei der Verwendung von Hypobromit als Oxidationsmittel zweckmäßig, ein Disulfit, z.B. Natriumdisulfit, (Na₂S₂O₅) zuzusetzen, um Verfärbungen zu verhindern, die sonst durch Reaktion von überschüssigem Hypobromit mit der Säure unter Freisetzung von Brom auftreten können.

Die fluorierten Chloracetophenone (α-Chloracetophenone und α,α-Dichloracetophenone) der Formel IIa 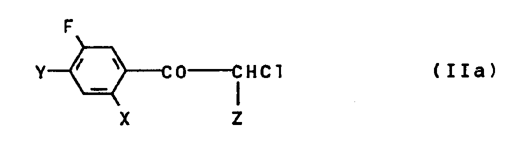
worin entweder a):
- Z =: Cl und
- X =: Br, Cl, F,
- Y =: Cl, F und

einer der Reste X oder Y auch Wasserstoff bedeuten, oder b):
- Z =: H und
- X =: Cl und
- Y =: F, oder
- X =: Br und
- Y =: Cl, F oder H bedeuten,

sind neu und werden im Rahmen der vorliegenden Erfindung beansprucht. Bevorzugt sind solche Verbindungen der Formel IIa, bei denen Y Fluor und X Fluor oder Chlor und Z Wasserstoff oder insbesondere Chlor bedeuten. Die Verbindungen der Formeln II und IIa werden in hervorragenden Ausbeuten dadurch erhalten, daß eine Verbindung der Formel III 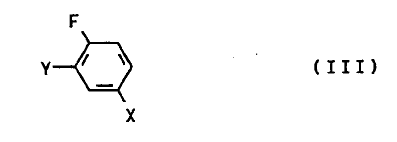
worin
X und Y die im Zusammenhang mit den Verbindungen der Formeln II und IIa angegebenen Bedeutungen besitzen, mit Mono- oder Dichloracetylchlorid in Gegenwart eines Acylierungskatalysators umgesetzt wird. Die Umsetzung mit Monochloracetylchlorid liefert die Verbindungen der Formeln II und IIa mit Z = Wasserstoff, und die Umsetzung mit Dichloracetylchlorid liefert die Verbindungen der Formeln II und IIa mit Z = Chlor, jeweils in hervorragenden Ausbeuten. In beiden Fällen kann die Umsetzung normalerweise bei einer Temperatur von 20 bis 100°C durchgeführt werden. Die Anwendung von Temperaturen über 100°C ist nicht erforderlich. Vorzugsweise wird die Umsetzung bei einer Temperatur von 20 bis 90°C und ganz besonders bevorzugt bei 20 bis 80°C durchgeführt. Bei der Umsetzung der Verbindungen der Formel III mit Dichloracetylchlorid können häufig Temperaturen von 20 bis 70°C zur Anwendung kommen. Die Reaktion kann auch in einem inerten Verdünnungsmittel durchgeführt werden, so z.B. in Nitrobenzol oder einem Halogenkohlenwasserstoff, wie z.B. Dichlorethan oder Trichlorethan oder in einem Gemisch inerter Verdünnungsmittel. Die Reaktion wird jedoch vorzugsweise ohne Verdünnungsmittel durchgeführt.

Als Acylierungskatalysator sind Lewis-Säuren, wie z.B. Aluminiumchlorid, Aluminiumbromid, Zinkchlorid, Bortrifluorid, Eisen(III)chlorid, Zinn(IV)chlorid, Titantetrachlorid und andere sogenannte Friedel-Crafts-Katalysatoren geeignet. Üblicherweise wird Aluminiumchlorid verwendet. Die genannten Acylierungskatalysatoren werden, wie üblich, in wasserfreier Form eingesetzt.

Die Fluorbenzole der Formel III sind bekannt bzw. können nach an sich bekannten Verfahren hergestellt werden. Mono- und Dichloracetylchlorid sind bekannte Verbindungen. Zweckmäßigerweise werden bei der Umsetzung Fluorbenzol der Formel III, Mono- oder Dichloracetylchlorid und Acylierungskatalysator im Molverhältnis 1 : (1 bis 2) : (1,2 bis 3), vorzugsweise 1 : (1 bis 1,6) : (1,3 bis 2,5) eingesetzt. Bei der Verwendung von Dichloracetylchlorid beträgt das Molverhältnis Fluorbenzol der Formel III : Dichloracetylchlorid : Acylierungskatalysator in vielen Fällen 1 : (1,1 bis 1,6) : (1,3 bis 2,1).

Die Verbindungen der Formel II werden in hervorragenden Ausbeuten von üblicherweise 89 % der Theorie und mehr unter milden Bedingungen erhalten und können in der Regel ohne weitere Reinigung bei der nachfolgenden Oxidation mit Hypohalogenit eingesetzt werden. Die erfindungsgemäße Umsetzung des 1,2,4-Trifluorbenzols mit Chloracetylchlorid zu 2,4,5-Trifluor-α-chloracetophenon verläuft schon bei milden Temperaturen in Ausbeuten von 89 bis 90% der Theorie. Bei der erfindungsgemäßen Umsetzung von 1-Chlor-3,4-difluorbenzol mit Chloracetylchlorid zu 2-Chlor-4,5-difluor-α-chloracetophenon werden bei milden Temperaturen sogar Ausbeuten von 95 % der Theorie und mehr erhalten. Dies war in hohem Maße überraschend, da z.B. bei der Umsetzung von 1-Chlor-3,4-difluorbenzol mit Acetylchlorid unter vergleichbaren Bedingungen 2-Chlor-4,5-difluoracetophenon nur in wesentlich geringeren Ausbeuten erhalten werden kann und 1,2,4-Trifluorbenzol mit Trichloracetylchlorid unter vergleichbaren Bedingungen überhaupt nicht zu dem gewünschten α,α,α-Trichloracetophenon reagiert.

Durch die Verwendung von Dichloracetylchlorid, das bevorzugt ist, kann nicht nur die bei der Verwendung von Acetylchlorid bei der Umsetzung gleicher Fluorbenzole der Formel III erforderliche Temperatur z.T. erheblich abgesenkt werden, sondern es sind darüberhinaus oftmals auch nur geringere Überschüsse an Acylierungsmittel und/oder Acylierungskatalysator erforderlich. So wird bei dem Verfahren der EP-A2-303 291 1-Chlor-3,4-difluorbenzol mit Acetylchlorid in Gegenwart von Aluminiumchlorid im Molverhältnis 1 : 1,5 : 1,5 umgesetzt, während nach der vorliegenden Erfindung 1-Chlor-3,4-difluorbenzol mit Dichloracetylchlorid in Gegenwart von Aluminiumchlorid im Molverhältnis 1 : 1,2 : 1,5 (vgl. nachfolgendes Beispiel 6) umgesetzt werden kann, was eine Reduzierung der Menge des Acylierungsmittels um 20 % bedeutet.

Bei dem Verfahren der EP-A2-342 849 wird 1-Brom-3,4-difluorbenzol mit Acetylchlorid in Gegenwart von Aluminiumchlorid im Molverhältnis 1 : 1,5 : 2,5 umgesetzt, während nach dem Verfahren der vorliegenden Erfindung 1-Brom-3,4-difluorbenzol mit Dichloracetylchlorid in Gegenwart von Aluminiumchlorid im Molverhältnis 1 : 1,5 : 2,0 (vgl. nachfolgendes Beispiel 11) umgesetzt wird, was eine Reduzierung der Menge des Acylierungskatalysators um 20 % bedeutet.

Durch die vorliegende Erfindung ist es möglich, ausgehend von Verbindungen der Formel III fluorierte Benzoesäuren der Formel I in hervorragenden Ausbeuten und Reinheiten herzustellen, ohne daß im Verlaufe des Gesamtverfahrens Temperaturen über 100°C angewandt werden müssen. Das erfindungsgemäße Verfahren weist weiterhin z.B. den Vorteil auf, daß in der Seitenkette der Verbindungen der Formel II bereits mindestens ein Oxidationsäquivalent eingebracht worden ist, so daß die Hypohalogenit-Oxidation schonender und mit weniger Hypohalogenit durchgeführt werden kann, als dies in vergleichbaren Fällen bisher möglich war.

Bei einer anschließenden Umsetzung der erhaltenen Verbindungen der Formel II mit Hypohalogenit werden Ausbeuten an den fluorierten Benzoesäuren der Formel I erhalten, die, bezogen auf eingesetztes Fluorbenzol der Formel III, normalerweise bei 80 % der Theorie und darüber liegen.

In den nachfolgenden Beispielen wird die Erfindung weiter erläutert. Soweit nichts anderes angegeben ist, bedeuten Prozentangaben Gewichtsprozente. Das verwendete Aluminiumchlorid wird in wasserfreier Form eingesetzt. Die ¹H- und ¹⁹F-NMR Spektren wurden mit einem 60 MHz-Gerät aufgenommen.

### Beispiel 1:

### 2,4,5-Trifluor-α-chloracetophenon

In 99 g (0,75 mol) 1,2,4-Trifluorbenzol werden 239 g (1,79 mol) Aluminiumchlorid bei 20°C eingetragen. In die Suspension dosiert man unter Rühren bei 60°C innerhalb von 2 Stunden 130 g Chloracetylchlorid (1,15 mol). Man läßt 1 h bei 80°C nachreagieren und gießt anschließend das flüssige Reaktionsprodukt auf 1 l Eiswasser. Das Reaktionsprodukt wird dreimal mit insgesamt 300 ml Dichlormethan aus der wäßrigen Phase extrahiert. Die vereinigten organischen Phasen werden über Magnesiumoxid getrocknet und eingedampft. Anschließend wird das feste Rohprodukt über eine Vigreuxkolonne destilliert (Kp.: 104°C, 20 mbar).

Man erhält 139 g festes 2,4,5-Trifluor-α-chloracetophenon, das entspricht 89% der Theorie, bezogen auf 1,2,4-Trifluorbenzol.
- Fp.:: 71 - 73°C
- GC:: 98,5 %
- IR:: 1704 cm⁻¹ (〉C=0)
- GC/MS:: Molmassenpeak M⁺: ^{m}/e 208 (Hauptprodukt)
M⁺ - ClCH₂ = ^{m}/e 159
M⁺ - (ClCH₂-CO) = ^{m}/e 131

¹H-NMR (CDCl₃) δ ppm, bezogen auf TMS
6,9 - 7,4 ppm (1 H, m)
7,7 - 8,1 ppm (1 H, m)
4,7 - 4,8 ppm (2 H, s aufgespalten)
¹⁹F-NMR (CDCl₃) δ ppm, bezogen auf C₆F₆
-21,8 bis -22,8 ppm (1 F, m)
-38,9 bis -39,8 ppm (1 F, m)
-53 bis -54 ppm (1 F, m).

### Beispiel 2:

### 2,4,5-Trifluorbenzoesäure

Zu 846 g Natriumhypobromitlösung (12,7%ig an NaOBr) werden unter Rühren innerhalb von 2 h bei 30°C 62,6 g (0,3 mol) aufgeschmolzenes 2,4,5-Trifluor-α-chloracetophenon, hergestellt entsprechend Beispiel 1, zugetropft. Man rührt 30 min bei 30°C und anschließend 2 h bei 50°C nach.

Nach Abtrennen der organischen Phase wird die fast farblose wäßrige Phase mit 130 ml Salzsäure bei 50°C unter Zusatz von Natriumdisulfit auf pH 1 gebracht.

Auf diese Weise erhält man 48,0 g 2,4,5-Trifluorbenzoesäure, das sind 81,0 % der Theorie, bezogen auf 1,2,4-Trifluorbenzol unter Berücksichtigung der Acylierungsausbeute aus Beispiel 1.
Fp.: 96 - 98°C

### Beispiel 3:

### 2-Chlor-4,5-difluor-α-chloracetophenon

Zu 45,4 g (0,3 mol) 1-Chlor-3,4-difluorbenzol und 97,5 g (0,73 mol) Aluminiumchlorid läßt man innerhalb von 2 1/2 h bei 65°C 50,8 g (0,45 mol) Chloracetylchlorid zutropfen. Man rührt anschließend 1 h bei 65°C und weitere 1 1/2 h bei 80°C nach. Der Ansatz wird bei ca. 70°C auf 625 g Eiswasser gegossen. Man trennt bei Raumtemperatur die Phasen und wäscht die organische Phase mit 50 ml (2 x 25 ml) Wasser. Nach der Trennung erhält man 65,8 g flüssiges Produkt, das entspricht 95,7 % der Theorie. (GC-Gehalt: 96 %). Kp: 120 - 123°C (14,67 mbar)
- IR:: 1700 cm⁻¹ (〉C=0)
- GC/MS:: Molmassenpeak M⁺: ^{m}/e 224 (Hauptprodukt)
M⁺ - ClCH₂ = ^{m}/e 175 Basispeak
M⁺ - (ClCH₂-CO) = ^{m}/e 147

¹H-NMR (CDCl₃) δ ppm, bezogen auf TMS
4,8 ppm (2 H, s)
7,25 - 7,75 ppm (2 H, m)
¹⁹F-NMR (CDCl₃) δ ppm, bezogen auf C₆F₆
-24,7 bis -25,8 ppm (1 F, m)
-33,4 bis -34,4 ppm (1 F, m)

### Beispiel 4:

### 2-Chlor-4,5-difluorbenzoesäure

Zu 846 g Natriumhypobromitlösung (12,7%ig an NaOBr) werden unter Rühren innerhalb von 1 ¹/2 h bei 30°C 67,5 g (0,3 mol) 2-Chlor-4,5-difluor-α-chloracetophenon (hergestellt entsprechend Beispiel 3) zugetropft. Man rührt 30 min bei 30°C und anschließend 2 h bei 50°C nach.

Nach Abtrennen der organischen Phase wird die fast farblose wäßrige Phase mit 126 ml Salzsäure bei 50°C unter Zusatz von Natriumdisulfit auf pH 1 gestellt.

Auf diese Weise erhält man 52,0 g 2-Chlor-4,5-difluorbenzoesäure, das sind 86 % der Theorie, bezogen auf 1-Chlor-3,4-difluorbenzol. Fp.: 103 - 105°C.

### Beispiel 5

### 3,4-Difluor-α-chloracetophenon

566 g (4,24 mol) Aluminiumchlorid werden in 228 g (2 mol) 1,2-Difluorbenzol unter Rühren vorgelegt. Bei 35°C läßt man innerhalb von 2 1/2 h 226 g (2 mol) Chloracetylchlorid zutropfen. Man rührt 20 min nach und erwärmt anschließend 1 h bei 40°C. Der Ansatz wird dann in 2000g Eiswasser hydrolysiert. Daraufhin trennt man die organische Phase bei 40°C ab. Die organische Phase wird bei Raumtemperatur fest und weist nach GC/MS einen Gehalt von 99 % auf.

Die Ausbeute beträgt 364 g 3,4-Difluor-α-chloracetophenon, das entspricht 95,5 % d.Th., bezogen auf 1,2-Difluorbenzol.

GC/MS:
- Molmassenpeak M⁺:: ^{m}/e 190
- M⁺ - (ClCH₂) =: ^{m}/e 141
- M⁺ - (ClCH₂-CO) =: ^{m}/e 113

Das 3,4-Difluor-α-chloracetophenon kann analog den Beispielen 2 und 4 zur 3,4-Difluorbenzoesäure umgesetzt werden.

### Beispiel 6

### 2-Chlor-4,5-difluor-α,α-dichloracetophenon

In 74,3 g (0,5 mol) 1-Chlor-3,4-difluorbenzol trägt man bei Raumtemperatur unter Rühren auf einmal 100 g (0,75 mol) wasserfreies Aluminiumchlorid ein. Man erwärmt die Suspension auf 65°C und tropft unter Feuchtigkeitsausschluß 89 g (0,6 mol) Dichloracetylchlorid innerhalb von 3 h zu. Die Temperatur wird bei 65°C gehalten. Man läßt 4 h bei 65°C nachreagieren und gießt das olivgrüne flüssige Reaktionsgemisch anschließend zur Hydrolyse in 1 l Wasser. Dabei wird gut gerührt, und man läßt die Temperatur bis ca. 60°C ansteigen. Die organische Phase wird im Scheidetrichter bei ca. 50°C abgetrennt. Man erhält 125 g 2-Chlor-4,5-difluor-α,α-dichloracetophenon, das entspricht 96,4 % der Theorie, bezogen auf 1-Chlor-3,4-difluorbenzol.
- Gehalt (aus GC/MS): = 91 %
- MS: Molmassenpeak M⁺: = ^{m}/e 258 (260)
- M⁺ - CO-³⁵Cl: = ^{m}/e 195
- M⁺ - CHCl₂: = ^{m}/e 175 Basispeak
- M⁺ - (CHCl₂-CO): = ^{m}/e 174

¹⁹F-NMR (CDCl₃) δ ppm, bezogen auf C₆F₆
-25,5 ppm bis -26,6 ppm (1 F, m)
-34,6 ppm bis -35,6 ppm (1 F, m)

### Beispiel 7

### 2-Chlor-4,5-difluorbenzoesäure

Zu 1258 g Natriumhypobromitlösung (hergestellt aus 114 g Natriumhydroxid und 984 g H₂O unter Zugabe von 160 g Brom bei 0 bis 10°C) werden unter Rühren innerhalb von ca. 2 h bei 30°C 125 g 2-Chlor-4,5-difluor-α,α-dichloracetophenon aus Beispiel 6 zugetropft. Man rührt 45 min bei 30°C und anschließend 3 h bei 50°C nach.

Nach Abtrennen der organischen Phase wird die wäßrige Phase bei ca. 40°C mit 140 ml Salzsäure (32%ig) unter Zusatz von Natriumdisulfit auf pH 1 gebracht.

Auf diese Weise erhält man 83 g 2-Chlor-4,5-difluorbenzoesäure, das entspricht 86 % der Theorie, bezogen auf 1-Chlor-3,4-difluorbenzol im Beispiel 6.
Gehalt (aus HPLC) = 99 %,
Fp.: 103 bis 105°C.

### Beispiel 8

### 2-Chlor-4,5-difluorbenzoesäure

Zu 1000 g Natriumhypochloritlösung (12%ig an NaOCl) werden unter Rühren innerhalb von 1,5 h bei 60°C 125 g 2-Chlor-4,5-difluor-α,α-dichloracetophenon, hergestellt entsprechend Beispiel 6, zugetropft. Man rührt 30 min bei 60°C nach und erwärmt für 2 ¹/2 h zum Sieden, dabei wird die entstehende organische Phase über einen Flüssigkeitsabscheider abgetrennt.

Die wäßrige Phase wird bei ca. 40°C mit 80 ml Salzsäure (32%ig) unter Zusatz von Natriumdisulfit auf pH 1 gebracht. Nach üblicher Isolierung und Trocknung erhält man 82 g 2-Chlor-4,5-difluorbenzoesäure, das entspricht 85 % der Theorie, bezogen auf 1-Chlor-3,4-difluorbenzol. Gehalt (aus HPLC) = 99,3 %

### Beispiel 9

### 2,4,5-Trifluor-α,α-dichloracetophenon

Eine Suspension aus 66,1 g (0,5 mol) 1,2,4-Trifluorbenzol und 133,4 g (1 mol) Aluminiumchlorid wird auf 55°C erwärmt. Bei dieser Temperatur dosiert man unter Feuchtigkeitsausschluß und unter Rühren innerhalb von 2 h 110 g (0,75 mol) Dichloracetylchlorid zu der Suspension. Es entwickelt sich ein kontinuierlicher HCl-Gasstrom.

Zur Vervollständigung der Reaktion läßt man ca. 2 h bei 60°C nachreagieren. Die HCl-Gasentwicklung läßt gegen Ende der Reaktion deutlich nach. Das flüssige Reaktionsgemisch wird zur Hydrolyse in 1 l Wasser eingegossen. Dabei wird gut durchgerührt. Nach vollendeter Hydrolyse wird die organische Phase bei 40°C abgetrennt.

Ohne weitere Zwischenaufreinigung erhält man 116 g der Titelverbindung, das entspricht 95,5 % der Theorie. Gehalt (aus GC/MS) = 95 %.
- MS: Molmassenpeak M⁺: = ^{m}/e 242 (244)
- M⁺ - CO-Cl: = ^{m}/e 179
- M⁺ - CHCl₂: = ^{m}/e 159
- M⁺ - (CHCl₂CO): = ^{m}/e 131

¹⁹F-NMR (CDCl₃) δ ppm, bezogen auf C₆F₆
-22,4 ppm bis -23,4 ppm (1 F, m)
-40,4 ppm bis -41,4 ppm (1 F, m)
-53,7 ppm bis -54,7 ppm (1 F, m)

### Beispiel 10

### 2,4,5-Trifluorbenzoesäure

Zu 1258 g Natriumhypobromitlösung (9,5 %ig an NaOBr, entsprechend 1 mol) werden unter Rühren innerhalb von ca. 2 h bei 30°C 116 g 2,4,5-Trifluor-α,α-dichloracetophenon (aus Beispiel 9) zugetropft. Man rührt 30 min bei 30°C und anschließend 3 h bei 50°C nach. Nach Abtrennen der organischen Phase wird die wäßrige Phase mit 250 ml Wasser verdünnt und mit 145 ml Salzsäure (32%ig) bei 40°C unter Zusatz von Natriumdisulfit auf pH 1 gebracht.

Auf diese Weise erhält man 72 g 2,4,5-Trifluorbenzoesäure, das entspricht 82 % der Theorie, bezogen auf 1,2,4-Trifluorbenzol in Beispiel 9.
Gehalt (aus HPLC) = 99 %.
Fp.: 96 bis 98°C

### Beispiel 11

### 2-Brom-4,5-difluor-α,α-dichloracetophenon

Zu 96,5 g (0,5 mol) 3,4-Difluorbrombenzol werden unter Rühren bei Raumtemperatur 133,4 g (1 mol) Aluminiumchlorid gegeben. Die Suspension wird auf 70°C erwärmt und unter Feuchtigkeitsausschluß innerhalb von 2 h mit 110 g (0,75 mol) Dichloracetylchlorid tropfenweise versetzt. Zur Vervollständigung der Reaktion wird 1 h bei 70°C nachgerührt. Nach Beendigung der Reaktion wird das flüssige Reaktionsgemisch zur Hydrolyse in 1 l Wasser eingegossen. Dabei wird gut gerührt, und man läßt die Temperatur bis ca. 60°C ansteigen. Die organische Phase wird bei ca. 50°C abgetrennt.

Man erhält 151 g 2-Brom-4,5-difluor-α,α-dichloracetophenon, das entspricht 99 % der Theorie.
- Gehalt (aus GC/MS): = 80 %
- MS: Molmassenpeak M⁺: = ^{m}/e 302/304
- M⁺ - CO-Cl: = ^{m}/e 239/241
- M⁺ - CHCl₂: = ^{m}/e 219 Basispeak /^{m}/e 221
- M⁺ - (CHCl₂CO): = ^{m}/e 191/193

¹⁹F-NMR (CDCl₃) δ ppm, bezogen auf C₆F₆
-25,8 ppm bis -26,6 ppm (1 F, m)
-33,5 ppm bis -34,5 ppm (1 F, m)

### Beispiel 12

### 2-Brom-4,5-difluorbenzoesäure

Zu 1258 g Natriumhypobromitlösung (9,5 %ig an NaOBr, entsprechend 1 mol) werden unter Rühren innerhalb von ca. 1 1/4 h bei 30°C 151 g 2-Brom-4,5-difluor-α,α-dichloracetophenon zugetropft. Man rührt 30 min bei 30°C und anschließend 3 h bei 50°C nach.

Nach Abtrennen der organischen Phase wird die wäßrige Phase bei ca. 40°C mit 125 ml Salzsäure (32%ig) unter Zusatz von Natriumdisulfit auf pH 1 gebracht. Nach üblicher Isolierung erhält man 101 g 2-Brom-4,5-difluorbenzoesäure, das entspricht 85 % der Theorie, bezogen auf 1-Brom-3,4-difluorbenzol in Beispiel 11.
Gehalt (aus HPLC) = 94 %
Schmelzpunkt Fp.: 108 bis 111°C.

### Beispiel 13

### 4-Chlor-2,5-difluor-α,α-dichloracetophenon

74,3 g (0,5 mol) 1-Chlor-2,5-difluorbenzol und 133,4 g (1 mol) Aluminiumchlorid werden entsprechend Beispiel 6 bei 65°C zur Reaktion gebracht. Man tropft unter Feuchtigkeitsausschluß 89 g (0,6 mol) Dichloracetylchlorid innerhalb von 3 h zu und läßt 2 h bei 70°C nachrühren, so daß die Gesamtreaktionszeit 4,5 h beträgt. Es wird in 1 l Wasser hydrolisiert und die organische Phase bei 40°C im Scheidetrichter abgetrennt. Man erhält 124 g flüssiges Reaktionsprodukt, das sind 95 % der Theorie, bezogen auf 1-Chlor-2,5-difluorbenzol.
Gehalt (aus GC/MS) = 93 %
- MS: Molmassenpeak M⁺: = ^{m}/e 258 (260)
- M⁺ - CO-Cl: = ^{m}/e 195
- M⁺ - CHCl₂: = ^{m}/e 175 Basispeak
- M⁺ - (CHCl₂CO): = ^{m}/e 147

¹⁹F-NMR (CDCl₃) δ ppm, bezogen auf C₆F₆
-43,7 ppm bis -44,6 ppm (1 F, m)
-50,6 ppm bis -51,4 ppm (1 F, m)

### Beispiel 14

### 4-Chlor-2,5-difluor-benzoesäure

Zu 1258 g Natriumhypobromitlösung (9,5 %ig) werden innerhalb von 1 h bei 30°C 124 g 4-Chlor-2,5-difluor-α,α-dichloracetophenon aus Beispiel 13 unter Rühren zugetropft. Man rührt 45 min bei 30°C und anschließend 3 h bei 50°C nach.

Nach Verdünnen mit 1500 ml Wasser wird der Ansatz mit 70 ml Salzsäure (32 %) unter Zusatz von Natriumdisulfit auf pH 6 gestellt und die organische Phase abgetrennt. Anschließend wird mit 50 ml Salzsäure (32 %) unter Zusatz von Natriumdisulfit die Benzoesäure bis pH 1 gefällt. Auf diese Weise erhält man 77,3 g 4-Chlor-2,5-difluorbenzoesäure, das entspricht 80,3 % der Theorie, bezogen auf 0,5 mol eingesetztes 1-Chlor-2,5-difluorbenzol in Beispiel 13.
Gehalt (aus HPLC): 99,5 %
Fp.: 153 bis 155°C

### Beispiel 15

### 3,4-Difluor-α,α-dichloracetophenon

57 g (0,5 mol) 1,2-Difluorbenzol und 93,4 g (0,7 mol) Aluminiumchlorid werden bei 30°C unter Feuchtigkeitsausschluß mit 88,4 g (0,6 mol) Dichloracetylchlorid unter Rühren zur Reaktion gebracht. Einschließlich Dosierzeit wird insgesamt 3 h bei 30°C erwärmt. Gegen Ende der Reaktion läßt die HCl-Entwicklung deutlich nach. Das Reaktionsgemisch gießt man zur Hydrolyse unter Rühren in 1 l Eiswasser. Das flüssige Produkt wird im Scheidetrichter bei 25°C abgetrennt.

Man erhält 105 g der Titelverbindung, das entspricht 93 % der Theorie, bezogen auf 1,2-Difluorbenzol.
Gehalt (aus GC/MS) = 96 %
- MS: Molmassenpeak M⁺: = ^{m}/e 224 (226) mit ³⁵Cl
- M⁺ - CO-³⁵Cl: = ^{m}/e 161
- M⁺ - CHCl₂: = ^{m}/e 141 Basispeak
- M⁺ - (CHCl₂CO): = ^{m}/e 113

¹⁹F-NMR (CDCl₃) δ ppm, bezogen auf C₆F₆
-27,1 ppm bis -28,1 ppm (1 F, m)
-35,4 ppm bis -36,6 ppm (1 F, m)

### Beispiel 16

### 3,4-Difluorbenzoesäure

Das in Beispiel 15 erhaltene 3,4-Difluor-α,α-dichloracetophenon wird ohne Zwischenaufreinigung mit Natriumhypobromitlösung gemäß der Arbeitsweise in Beispiel 10 zur 3,4-Difluorbenzoesäure oxidiert.

Man erhält 68 g der Titelverbindung, das entspricht 86 % der Theorie, bezogen auf eingesetztes 1,2-Difluorbenzol in Beispiel 15.
Gehalt (aus HPLC) = 99 %
Fp.: 120 bis 122°C.

### Beispiel 17

### 2,5-Difluor-α,α-dichloracetophenon

Eine Suspension aus 57 g (0,5 mol) 1,4-Difluorbenzol und 93,4 g (0,7 mol) wasserfreiem Aluminiumtrichlorid wird auf 45°C erwärmt. Bei dieser Temperatur dosiert man unter Feuchtigkeitsausschluß und unter Rühren innerhalb von 2 h 88,4 g (0,6 mol) Dichloracetylchlorid zu der Suspension.

Zur Vervollständigung der Reaktion läßt man 1 h bei 45°C nachreagieren. Nach Beendigung der Reaktion wird das flüssige Reaktionsgemisch zur Hydrolyse in 1 l Wasser eingegossen. Dabei wird gut gerührt und man läßt die Temperatur bis 40°C ansteigen. Die organische Phase wird bei ca. 40°C scharf abgetrennt. Man erhält 96 g 2,5-Difluor-α,α-dichloracetophenon, das entspricht 85,3 % der Theorie, bezogen auf eingesetztes 1,4-Difluorbenzol.
Gehalt (aus GC/MS): 93,5 % (6,1 % 1,4-Difluorbenzol)
- MS: Molmassenpeak M⁺: = ^{m}/e 224 (226) mit ³⁵Cl
- M⁺ - CO-³⁵Cl: = ^{m}/e 161
- M⁺ - CHCl₂: = ^{m}/e 141 Basispeak
- M⁺ - (CHCl₂CO): = ^{m}/e 113

¹⁹F-NMR (CDCl₃) δ ppm, bezogen auf C₆F₆
-45,7 ppm bis -46,6 ppm (1 F, m)
-48 ppm bis -48,9 ppm (1 F, m)

### Beispiel 18

### 2,5-Difluorbenzoesäure

Zu 1258 g Natriumhypobromitlösung (9,5 %ig) werden unter Rühren innerhalb von ca. 1 1/4 h bei 30°C 96 g 2,5-Difluor-α,α-dichloracetophenon aus Beispiel 17 zugetropft. Man rührt 45 min bei 30°C und anschließend 3 h bei 50°C nach.

Nach Verdünnen mit 250 ml Wasser wird der Ansatz mit 75 ml Salzsäure (32 %) unter Zusatz von Natriumdisulfit auf pH 6 gestellt und die organische Phase abgetrennt. Anschließend wird mit 50 ml Salzsäure (32 %) unter Zusatz von Natriumdisulfit auf pH 1 gestellt.

Man erhält 63,6 g 2,5-Difluorbenzoesäure, das entspricht 80,5 % d.Th., bezogen auf 0,5 mol eingesetztes 1,4-Difluorbenzol in Beispiel 17.
Gehalt (aus HPLC): 99,6 %,
Schmelzpunkt Fp.: 128 bis 130°C.

## Patentansprüche (Patentansprüche für folgende(n) Vertragsstaat(en): DE, GB, FR, IT, LI, CH, BE, DK)

1. Verfahren zur Herstellung von fluorierten Benzoesäuren der Formel I 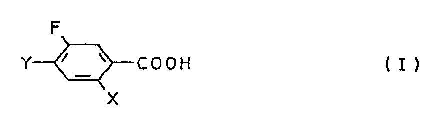 worin
X = Br, Cl, F,
Y = Cl, F
und einer der Reste X oder Y auch Wasserstoff bedeuten, dadurch gekennzeichnet, daß ein fluoriertes Chloracetophenon der Formel II 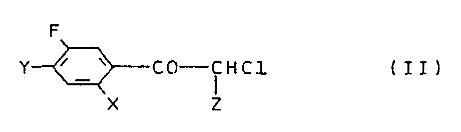 worin
X = Br, Cl, F,
Y = Cl, F,
Z = H oder Cl und
einer der Reste X oder Y auch Wasserstoff bedeuten, mit einem Hypohalogenit umgesetzt wird.

2. Verfahren nach Anspruch 1, dadurch gekennzeichnet, daß die Verbindung der Formel II mit Natriumhypobromit umgesetzt wird.

3. Verfahren nach Anspruch 1 und/oder 2, dadurch gekennzeichnet, daß die Umsetzung bei einer Temperatur von 0°C bis 100°C, vorzugsweise von 10 bis 80°C, und ganz besonders bevorzugt von 20 bis 60°C durchgeführt wird.

4. Verfahren nach einem oder mehreren der Ansprüche 1 bis 3, dadurch gekennzeichnet, daß eine Verbindung der Formel III 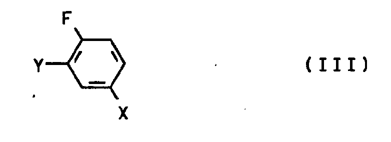 worin
X = Br, Cl, F,
Y = Cl, F
und einer der Reste X oder Y auch Wasserstoff bedeuten, mit Mono- oder Dichloracetylchlorid in Gegenwart eines Acylierungskatalysators zu einer Verbindung der Formel II umgesetzt und diese, gegebenenfalls nach ihrer Isolierung, mit dem Hypohalogenit umgesetzt wird.

5. Verfahren nach Anspruch 4, dadurch gekennzeichnet, daß die Umsetzung der Verbindung III mit Mono- oder Dichloracetylchlorid bei einer Temperatur von 20 bis 100°C, insbesondere von 20 bis 90°C, bevorzugt von 20 bis 80°C, und besonders bevorzugt von 20 bis 70°C durchgeführt wird.

6. Verfahren nach einem oder mehreren der Ansprüche 1 bis 5, dadurch gekennzeichnet, daß eine Verbindung der Formel II bzw. III eingesetzt wird, bei der Y Fluor und X Fluor oder Chlor bedeuten.

7. Fluorierte Chloracetophenone der Formel IIa 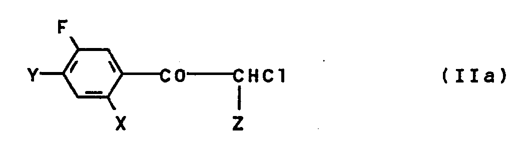 worin entweder a):
Z = Cl und
X = Br, Cl, F,
Y = Cl, F und
einer der Reste X oder Y auch Wasserstoff bedeuten, oder b):
Z = H und
X = Cl und
Y = F, oder
X = Br und
Y = Cl, F oder H bedeuten.

8. Fluorierte Chloracetophenone nach Anspruch 7, dadurch gekennzeichnet, daß Z Wasserstoff bedeutet.

9. Fluorierte Chloracetophenone nach Anspruch 7, dadurch gekennzeichnet, daß Z Chlor bedeutet.

10. Fluorierte Chloracetophenone nach Anspruch 7, dadurch gekennzeichnet, daß Y Fluor und X Fluor oder Chlor bedeuten.

11. Verfahren zur Herstellung der fluorierten Chloracetophenone der Formel IIa 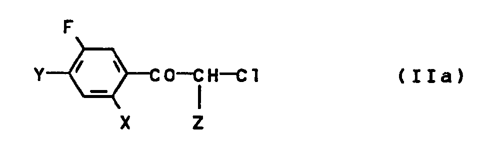 worin entweder a):
Z = Cl und
X = Br, Cl, F,
Y = Cl, F und
einer der Reste X oder Y auch Wasserstoff bedeuten oder b):
Z = H und
X = Cl oder F und
Y = F, oder
X = Br und
Y = Cl, F oder H
bedeuten, dadurch gekennzeichnet, daß a) für den Fall, daß eine Verbindung der Formel II mit Z = Cl hergestellt werden soll, eine Verbindung der Formel III 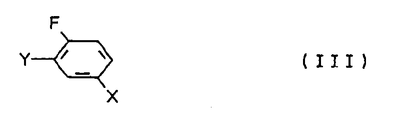 worin
X = Br, Cl, F,
Y = Cl, F
und einer der Reste X oder Y auch Wasserstoff bedeuten, mit Dichloracetylchlorid in Gegenwart eines Acylierungskatalysators umgesetzt wird,
oder daß b) für den Fall, daß eine Verbindung der Formel II mit Z = H hergestellt werden soll, eine Verbindung der Formel III 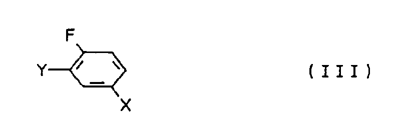 worin
X = Cl oder F und
Y = F, oder
X = Br und
Y = Cl, F oder H
bedeuten, mit Monochloracetylchlorid in Gegenwart eines Acylierungskatalysators umgesetzt wird.

12. Verfahren nach Anspruch 11, dadurch gekennzeichnet, daß die Umsetzung bei einer Temperatur von 20 bis 100°C, insbesondere von 20 bis 90°C, und bevorzugt von 20 bis 80°C durchgeführt wird.

13. Verfahren nach Anspruch 11 und/oder 12, dadurch gekennzeichnet, daß eine Verbindung der Formel III mit Dichloracetylchlorid bei einer Temperatur von 20 bis 70°C umgesetzt wird.

## Patentansprüche (Patentansprüche für folgende(n) Vertragsstaat(en): ES)

1. Verfahren zur Herstellung von fluorierten Benzoesäuren der Formel I 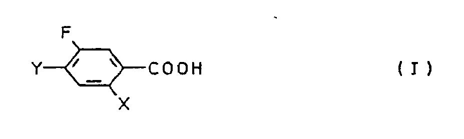 worin
X = Br, Cl, F,
Y = Cl, F
und einer der Reste X oder Y auch Wasserstoff bedeuten, dadurch gekennzeichnet, daß ein fluoriertes Chloracetophenon der Formel II 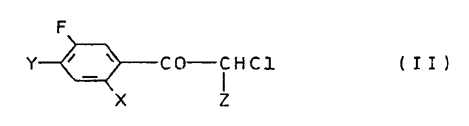 worin
X = Br, Cl, F,
Y = Cl, F,
Z = H oder Cl und
einer der Reste X oder Y auch Wasserstoff bedeuten, mit einem Hypohalogenit umgesetzt wird.

2. Verfahren nach Anspruch 1, dadurch gekennzeichnet, daß die Verbindung der Formel II mit Natriumhypobromit umgesetzt wird.

3. Verfahren nach Anspruch 1 und/oder 2, dadurch gekennzeichnet, daß die Umsetzung bei einer Temperatur von 0°C bis 100°C, vorzugsweise von 10 bis 80°C, und ganz besonders bevorzugt von 20 bis 60°C durchgeführt wird.

4. Verfahren nach einem oder mehreren der Ansprüche 1 bis 3, dadurch gekennzeichnet, daß eine Verbindung der Formel III 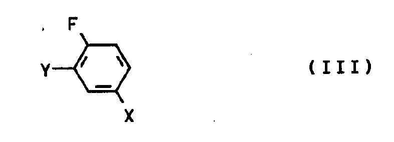 worin
X = Br, Cl, F,
Y = Cl, F
und einer der Reste X oder Y auch Wasserstoff bedeuten, mit Mono- oder Dichloracetylchlorid in Gegenwart eines Acylierungskatalysators zu einer Verbindung der Formel II umgesetzt und diese, gegebenenfalls nach ihrer Isolierung, mit dem Hypohalogenit umgesetzt wird.

5. Verfahren nach Anspruch 4, dadurch gekennzeichnet, daß die Umsetzung der Verbindung III mit Mono- oder Dichloracetylchlorid bei einer Temperatur von 20 bis 100°C, insbesondere von 20 bis 90°C, bevorzugt von 20 bis 80°C, und besonders bevorzugt von 20 bis 70°C durchgeführt wird.

6. Verfahren nach einem oder mehreren der Ansprüche 1 bis 5, dadurch gekennzeichnet, daß eine Verbindung der Formel II bzw. III eingesetzt wird, bei der Y Fluor und X Fluor oder Chlor bedeuten.

7. Verfahren zur Herstellung fluorierter Chloracetophenone der Formel IIa 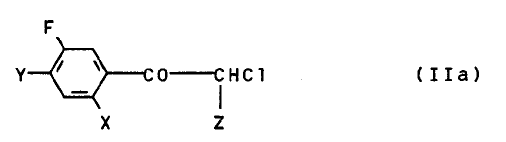 worin entweder a):
Z = Cl und
X = Br, Cl, F,
Y = Cl, F und
einer der Reste X oder Y auch Wasserstoff bedeuten oder b):
Z = H und
X = Cl oder F und
Y = F, oder
X = Br und
Y = Cl, F oder H
bedeuten, dadurch gekennzeichnet, daß a) für den Fall, daß eine Verbindung der Formel II mit Z = Cl hergestellt werden soll, eine Verbindung der Formel III 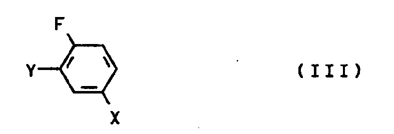 worin
X = Br, Cl, F,
Y = Cl, F
und einer der Reste X oder Y auch Wasserstoff bedeuten, mit Dichloracetylchlorid in Gegenwart eines Acylierungskatalysators umgesetzt wird,
oder daß b) für den Fall, daß eine Verbindung der Formel II mit Z = H hergestellt werden soll, eine Verbindung der Formel III 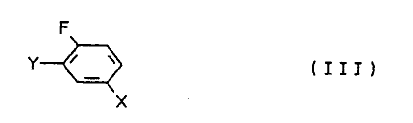 worin
X = Cl oder F und
Y = F oder
X = Br und
Y = Cl, F oder H
bedeuten, mit Monochloracetylchlorid in Gegenwart eines Acylierungskatalysators umgesetzt wird.

8. Verfahren nach Anspruch 7, dadurch gekennzeichnet, daß die Umsetzung bei einer Temperatur von 20 bis 100°C, insbesondere von 20 bis 90°C, und bevorzugt von 20 bis 80°C durchgeführt wird.

9. Verfahren nach Anspruch 7 und/oder 8, dadurch gekennzeichnet, daß eine Verbindung der Formel III mit Dichloracetylchlorid bei einer Temperatur von 20 bis 70°C umgesetzt wird.

10. Verfahren nach einem oder mehreren der Ansprüche 7 bis 9, dadurch gekennzeichnet, daß Monochloracetylchlorid eingesetzt wird.

11. Verfahren nach einem oder mehreren der Ansprüche 7 bis 9, dadurch gekennzeichnet, daß Dichloracetylchlorid eingesetzt wird.

12. Verfahren nach einem oder mehreren der Ansprüche 7 bis 11, dadurch gekennzeichnet, daß eine Verbindung der Formel III eingesetzt wird, bei der Y Fluor und X Fluor oder Chlor bedeuten.

## Claims (Claims for the following Contracting State(s): DE, GB, FR, IT, LI, CH, BE, DK)

1. Process for the preparation of fluorinated benzoic acids of the formula I 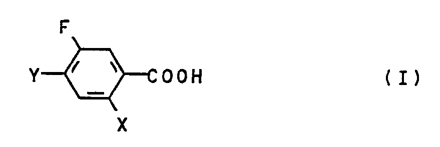 in which
X denotes Br, Cl or F,
Y denotes Cl or F
and one of the radicals X or Y also denotes hydrogen, characterized in that a fluorinated chloroacetophenone of the formula II 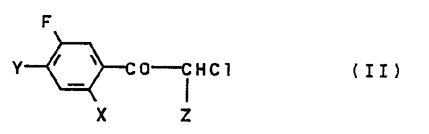 in which
X denotes Br, Cl or F,
Y denotes Cl or F,
Z denotes H or Cl and
one of the radicals X or Y also denotes hydrogen, is reacted with a hypohalite.

2. Process according to Claim 1, characterized in that the compound of the formula II is reacted with sodium hypobromite.

3. Process according to Claim 1 and/or 2, characterized in that the reaction is carried out at a temperature of 0°C to 100°C, preferably 10 to 80°C, and very particularly preferably 20 to 60°C.

4. Process according to one or more of Claims 1 to 3, characterized in that a compound of the formula III 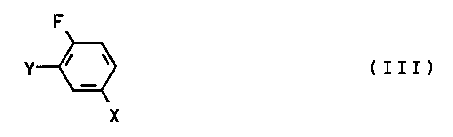 in which
X denotes Br, Cl or F,
Y denotes Cl or F
and one of the radicals X or Y also denotes hydrogen, is reacted with mono- or dichloroacetyl chloride in the presence of an acylating catalyst to give a compound of the formula II and this, if appropriate after its isolation, is reacted with hypohalite.

5. Process according to Claim 4, characterized in that the reaction of the compound III is carried out with mono- or dichloroacetyl chloride at a temperature of 20 to 100°C, in particular 20 to 90°C, preferably 20 to 80°C, and particularly preferably 20 to 70°C.

6. Process according to one or more of Claims 1 to 5, characterized in that a compound of the formula II or III is employed in which Y denotes fluorine and X denotes fluorine or chlorine.

7. Fluorinated chloroacetophenones of the formula IIa 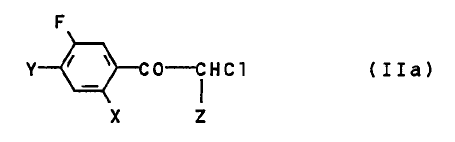 in which either a):
Z denotes Cl and
X denotes Br, Cl or F,
Y denotes Cl or F, and
one of the radicals X or Y also denotes hydrogen, or b):
Z denotes H and
X denotes Cl and
Y denotes F or
X denotes Br and
Y denotes Cl, F or H.

8. Fluorinated chloroacetophenones according to Claim 7, characterized in that Z denotes hydrogen.

9. Fluorinated chloroacetophenones according to Claim 7, characterized in that Z denotes chlorine.

10. Fluorinated chloroacetophenones according to Claim 7, characterized in that Y denotes fluorine and X denotes fluorine or chlorine.

11. Process for the preparation of the fluorinated chloroacetophenones of the formula IIa 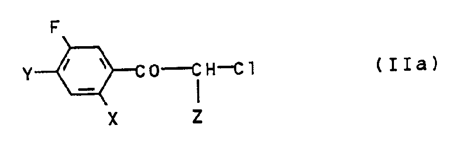 in which either a):
Z denotes Cl and
X denotes Br, Cl or F,
Y denotes Cl or F
and one of the radicals X or Y also denotes hydrogen, or b):
Z denotes H and
X denotes Cl or F and
Y denotes F, or
X denotes Br and
Y denotes Cl, F or H
characterized in that a) in case a compound of the formula II wherein Z denotes Cl is to be prepared, a compound of the formula III 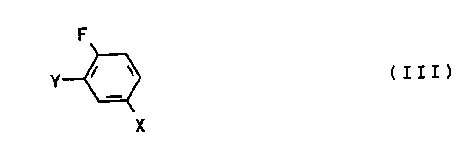 in which
X denotes Br, Cl, F,
Y denotes Cl, F
and one of the radicals X or Y denotes hydrogen, is reacted with dichloroacetyl chloride in the presence of an acylating catalyst,
or b): in case a compound of the formula II wherein Z denotes H is to be prepared, a compound of the formula III 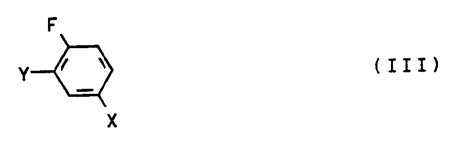 in which
X denotes Cl or F and
Y denotes F or
X denotes Br and
Y denotes Cl, F or H
is reacted with monochloroacetyl chloride in the presence of an acylating catalyst.

12. Process according to Claim 11, characterized in that the reaction is carried out at a temperature of 20 to 100°C, in particular 20 to 90°C, and preferably 20 to 80°C.

13. Process according to Claim 11 and/or 12, characterized in that a compound of the formula III is reacted with dichloroacetyl chloride at a temperature of 20 to 70°C.

## Claims (Claims for the following Contracting State(s): ES)

1. Process for the preparation of fluorinated benzoic acids of the formula I 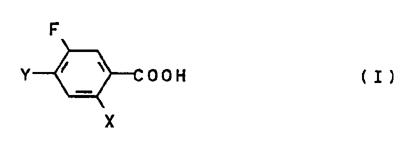 in which
X denotes Br, Cl or F,
Y denotes Cl or F
and one of the radicals X or Y also denotes hydrogen, characterized in that a fluorinated chloroacetophenone of the formula II 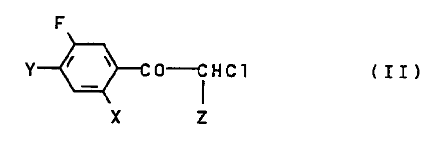 in which
X denotes Br, Cl or F,
Y denotes Cl or F,
Z denotes H or Cl and
one of the radicals X or Y also denotes hydrogen, is reacted with a hypohalite.

2. Process according to Claim 1, characterized in that the compound of the formula II is reacted with sodium hypobromite.

3. Process according to Claim 1 and/or 2, characterized in that the reaction is carried out at a temperature of 0°C to 100°C, preferably 10 to 80°C, and very particularly preferably 20 to 60°C.

4. Process according to one or more of Claims 1 to 3, characterized in that a compound of the formula III 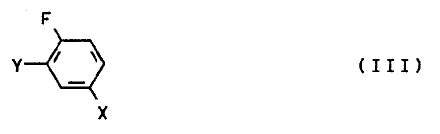 in which
X denotes Br, Cl or F,
Y denotes Cl or F
and one of the radicals X or Y also denotes hydrogen, is reacted with mono- or dichloroacetyl chloride in the presence of an acylating catalyst to give a compound of the formula II and this, if appropriate after its isolation, is reacted with hypohalite.

5. Process according to Claim 4, characterized in that the reaction of the compound III is carried out with mono- or dichloroacetyl chloride at a temperature of 20 to 100°C, in particular 20 to 90°C, preferably 20 to 80°C, and particularly preferably 20 to 70°C.

6. Process according to one or more of Claims 1 to 5, characterized in that a compound of the formula II or III is employed in which Y denotes fluorine and X denotes fluorine or chlorine.

7. Process for preparing fluorinated chloroacetophenones of the formula IIa 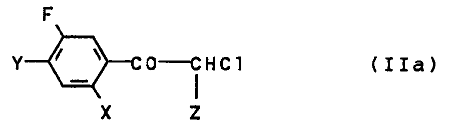 in which either a):
Z denotes Cl and
X denotes Br, Cl or F,
Y denotes Cl or F, and
one of the radicals X or Y also denotes hydrogen, or b):
Z denotes H and
X denotes Cl and
Y denotes F or
X denotes Br and
Y denotes Cl, F or H,
characterized in that a) in case a compound of the formula II wherein Z denotes Cl is to be prepared, a compound of the formula III 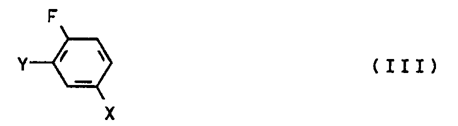 in which
X denotes Br, Cl, F,
Y denotes Cl, F
and one of the radicals X or Y denotes hydrogen, is reacted with dichloroacetyl chloride in the presence of an acylating catalyst,
or b): in case a compound of the formula II wherein Z denotes H is to be prepared, a compound of the formula III 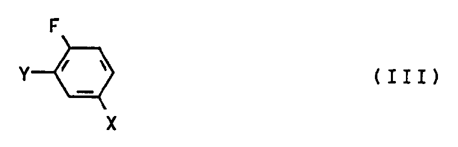 in which
X denotes Cl or F and
Y denotes F or
X denotes Br and
Y denotes Cl, F or H
is reacted with monochloroacetyl chloride in the presence of an acylating catalyst.

8. Process according to Claim 7, characterized in that the reaction is carried out at a temperature of 20 to 100°C, in particular 20 to 90°C, and preferably 20 to 80°C.

9. Process according to Claim 7 and/or 8, characterized in that a compound of the formula III is reacted with dichloroacetyl chloride at a temperature of 20 to 70°C.

10. Process according to one or more of Claims 7 to 9, characterized in that monochloroacetyl chloride is employed.

11. Process according to one or more of Claims 7 to 9, characterized in that dichloroacetyl chloride is employed.

12. Process according to one or more of Claims 7 to 11, characterized in that a compound of the formula III is employed in which Y denotes fluorine and X denotes fluorine or chlorine.

## Revendications (Revendications pour l'(les) Etat(s) contractant(s) suivant(s): DE, GB, FR, IT, LI, CH, BE, DK)

1. Procédé pour préparer des acides benzoïques fluorés de formule I 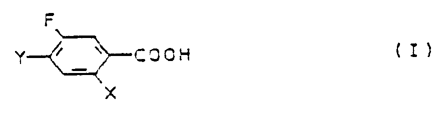 dans laquelle
X est Br, Cl, F,
Y est Cl, F et
l'un des radicaux X ou Y peut aussi représenter un hydrogène, caractérisé en ce qu'on fait réagir une chloracétophénone fluorée de formule II 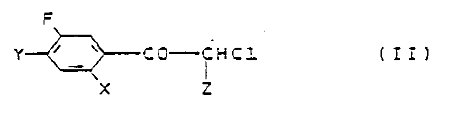 dans laquelle
X est Br, Cl, F,
Y est Cl, F,
Z est H ou Cl, et
l'un des radicaux X ou Y peut aussi être un hydrogène, avec un hypohalogénite.

2. Procédé selon la revendication 1, caractérisé en ce que le composé de formule II est mis à réagir avec de l'hypobromite de sodium.

3. Procédé selon la revendication 1 et/ou 2, caractérisé en ce que la réaction est mise en oeuvre à une température de 0 à 100°C, de préférence de 10 à 80°C et tout particulièrement de 20 à 60°C.

4. Procédé selon l'une ou plusieurs des revendications 1 à 3, caractérisé en ce qu'on fait réagir un composé de formule III 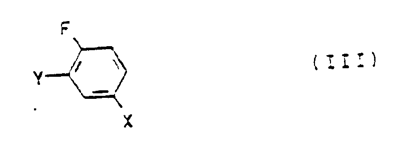 dans laquelle
X est Br, Cl, F,
Y est Cl, F
et l'un des radicaux X ou Y peut aussi être un hydrogène, avec du chlorure de mono- ou de dichloracétyle en présence d'un catalyseur d'acylation pour donner un composé de formule II, ce dernier, éventuellement après isolement, étant mis à réagir avec l'hypohalogénite.

5. Procédé selon la revendication 4, caractérisé en ce que la réaction du composé III avec le chlorure de mono- ou de dichloracétyle est mis en oeuvre à une température de 20 à 100°C, en particulier de 20 à 90°C, de préférence de 20 à 80°C et tout particulièrement de 20 à 70°C.

6. Procédé selon l'une ou plusieurs des revendications 1 à 5, caractérisé en ce qu'on utilise un composé de formule II ou III dans laquelle Y est le fluor et X est le fluor ou le chlore.

7. Chloracétophénones fluorées de formule IIa 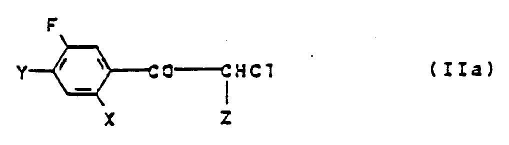 dans laquelle a) :
Z est Cl et
X est Br, Cl, F,
Y est Cl, F et
l'un des radicaux X ou Y peut aussi être un hydrogène, ou b) :
Z est H et
X est Cl et
Y est F, ou
X est Br, et
Y est Cl, F ou H.

8. Chloracétophénones fluorées selon la revendication 7, caractérisées en ce que Z est un hydrogène.

9. Chloracétophénones fluorées selon la revendication 7, caractérisées en ce que Z est le chlore.

10. Chloracétophénones fluorées selon la revendication 7, caractérisées en ce que Y est le fluor et X est le fluor ou le chlore.

11. Procédé pour préparer les chloracétophénones fluorées de formule IIa 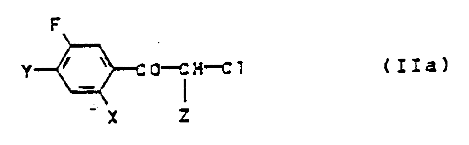 dans laquelle a) :
Z est Cl et
X est Br, Cl, F,
Y est Cl, F et
l'un des radicaux X ou Y peut aussi être un hydrogène, ou b) :
Z est H et
X est Cl et
Y est F, ou
X est Br, et
Y est Cl, F ou H,
caractérisé en ce que :
a) si l'on veut préparer un composé de formule II avec Z = Cl, on fait réagir un composé de formule III 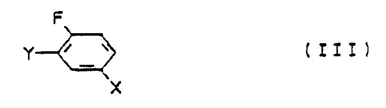 dans laquelle
X est Br, Cl, F,
Y est Cl, F
et l'un des radicaux X ou Y peut aussi être un hydrogène, avec du chlorure de dichloracétyle en présence d'un catalyseur d'acylation, ou bien,
b) si l'on veut préparer un composé de formule II avec Z = H, on fait réagir un composé de formule III 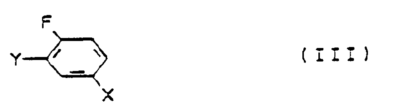 dans laquelle
X est Cl ou F, et
Y est F, ou
X est Br et
Y est Cl, F ou H
avec du chlorure de monochloracétyle en présence d'un catalyseur d'acylation.

12. Procédé selon la revendication 11, caractérisé en ce que la réaction est mise en oeuvre à une température de 20 à 100°C, en particulier de 20 à 90°C et tout particulièrement de 20 à 80°C.

13. Procédé selon la revendication 11 et/ou 12, caractérisé en ce qu'on fait réagir un composé de formule III avec du chlorure de dichloracétyle à une température de 20 à 70°C.

## Revendications (Revendications pour l'(les) Etat(s) contractant(s) suivant(s): ES)

1. Procédé pour préparer des acides benzoïques fluorés de formule I 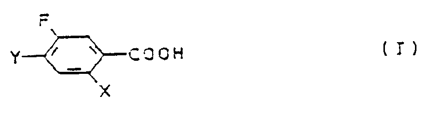 dans laquelle
X est Br, Cl, F,
Y est Cl, F et
l'un des radicaux X ou Y peut aussi représenter un hydrogène, caractérisé en ce qu'on fait réagir une chloracétophénone fluorée de formule II 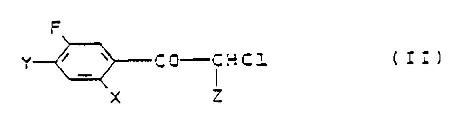 dans laquelle
X est Br, Cl, F,
Y est Cl, F,
Z est H ou Cl, et
l'un des radicaux X ou Y peut aussi être un hydrogène, avec un hypohalogénite.

2. Procédé selon la revendication 1, caractérisé en ce que le composé de formule II est mis à réagir avec de l'hypobromite de sodium.

3. Procédé selon la revendication 1 et/ou 2, caractérisé en ce que la réaction est mise en oeuvre à une température de 0 à 100°C, de préférence de 10 à 80°C et tout particulièrement de 20 à 60°C.

4. Procédé selon l'une ou plusieurs des revendications 1 à 3, caractérisé en ce qu'on fait réagir un composé de formule III 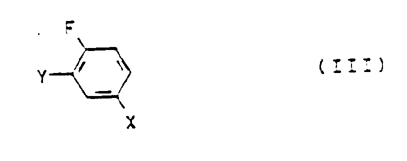 dans laquelle
X est Br, Cl, F,
Y est Cl, F
et l'un des radicaux X ou Y peut aussi être un hydrogène, avec du chlorure de mono- ou de dichloracétyle en présence d'un catalyseur d'acylation pour donner un composé de formule II, ce dernier, éventuellement après isolement, étant mis à réagir avec l'hypohalogénite.

5. Procédé selon la revendication 4, caractérisé en ce que la réaction du composé III avec le chlorure de mono- ou de dichloracétyle est mis en oeuvre à une température de 20 à 100°C, en particulier de 20 à 90°C, de préférence de 20 à 80°C et tout particulièrement de 20 à 70°C.

6. Procédé selon l'une ou plusieurs des revendications 1 à 5, caractérisé en ce qu'on utilise un composé de formule II ou III dans laquelle Y est le fluor et X est le fluor ou le chlore.

7. Procédé pour préparer des chloracétophénones fluorées de formule IIa 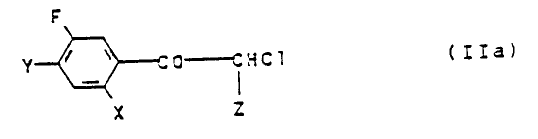 dans laquelle a) :
Z est Cl et
X est Br, Cl, F,
Y est Cl, F et
l'un des radicaux X ou Y peut aussi être un hydrogène, ou b) :
Z est H et
X est Cl et
Y est F, ou
X est Br, et
Y est Cl, F ou H,
caractérisé en ce que :
a) si l'on veut préparer un composé de formule II avec Z = Cl, on fait réagir un composé de formule III 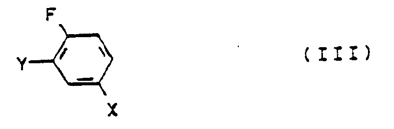 dans laquelle
X est Br, Cl, F,
Y est Cl, F
et l'un des radicaux X ou Y peut aussi être un hydrogène, avec du chlorure de dichloracétyle en présence d'un catalyseur d'acylation, ou bien,
b) si l'on veut préparer un composé de formule II avec Z = H, on fait réagir un composé de formule III 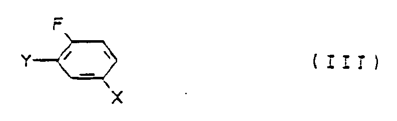 dans laquelle
X est Cl ou F, et
Y est F, ou
X est Br et
Y est Cl, F ou H
avec du chlorure de monochloracétyle en présence d'un catalyseur d'acylation.

8. Procédé selon la revendication 7, caractérisé en ce que la réaction est mise en oeuvre à une température de 20 à 100°C, en particulier de 20 à 90°C et tout particulièrement de 20 à 80°C.

9. Procédé selon la revendication 7 et/ou 8, caractérisé en ce qu'on fait réagir un composé de formule III avec du chlorure de dichloracétyle à une température de 20 à 70°C.

10. Procédé selon l'une ou plusieurs des revendications 7 à 9, caractérisé en ce qu'on utilise du chlorure de monochloracétyle.

11. Procédé selon l'une ou plusieurs des revendications 7 à 9, caractérisé en ce qu'on utilise du chlorure de dichloracétyle.

12. Procédé selon l'une ou plusieurs des revendications 7 à 11, caractérisé en ce qu'on utilise un composé de formule III dans laquelle Y est le fluor et X est le fluor ou le chlore.
